# EUROPEAN PATENT APPLICATION

(11) **EP 2 223 664 A1**
(43) Date of publication of application: **01.09.2010**
(21) Application number: 10001903.3
(22) Date of filing: 23.02.2010
(51) Int. Cl.: A61B 17/70

(54) **System and method for spinal stabilization**

(30) Priority: 24.02.2009 US 391523
(71) Applicant: Zimmer Spine, Inc., Minneapolis, MN 55439-2027 (US)
(72) Inventor: Todd, Ronald, C., Austin Texas 78730 (US)
(74) Representative: Manitz, Finsterwald & Partner GbR

(57) **Abstract**

Various stabilization rods and methods for manufacturing a connection member having a flexible section coupled between two rigid sections are disclosed. The rigid sections may have exterior surfaces adapted to have an increased surface area to inhibit the flexible section from separating from the rigid sections.

## Description

### TECHNICAL FIELD

The embodiments in this application are directed generally to medical stabilization implants and methods for implanting those devices, and more particularly to an implantable dynamic connection member with a flexible section coupled between rigid sections to provide selective support to the spine.

### BACKGROUND

A variety of surgical procedures on various parts of the human body can benefit from the use of various implantable stabilization devices. One such area is the human spine which consists of segments known as vertebrae linked by intervertebral disks and held together by ligaments. There are 24 movable vertebrae - 7 cervical (neck) vertebrae, 12 thoracic (chest) vertebrae, and 5 lumbar (back) vertebrae. Each vertebra has a somewhat cylindrical bony body (centrum), a number of wing-like projections (processes), and a bony arch. The arches are positioned so that the space they enclose forms the vertebral canal. The vertebral canal houses and protects the spinal cord, and within it the spinal fluid circulates. Ligaments and muscles are attached to various projections of the vertebrae. The bodies of the vertebrae form the supporting column of the skeleton. Fused vertebrae make up the sacrum and coccyx, the very bottom of the vertebral column.

Modern spine surgery often involves the use of spinal stabilization/fixation procedures, for example vertebral body fusion, to correct or treat various spine disorders and/or to support the spine. In conjunction with these procedures, spinal implants may be used to help stabilize the spine, correct deformities of the spine such as spondylolisthesis or pseudoarthrosis, facilitate fusion, treat spinal fractures, or for other purposes. Some spinal implants such as a spinal fixation system may provide fused and/or rigid support for the affected regions of the spine. These spinal stabilization systems often include one or more rods which can bear a portion of the forces that would otherwise be transmitted along the spine. These stabilization rods can be implanted along various regions of the spine and, in some procedures, can be attached to one or more vertebrae of the spine to provide support and stabilize, align, or otherwise treat the region of the spine of interest. The rods can be attached using a variety of anchor systems, including pedicle screws, hooks, cables, and other fixation devices. Examples of pedicle screws and other types of fixation systems and attachments are illustrated in U.S. Patent Nos. 4,763,644; 4,805,602; 4,887,596; 4,950,269; 5,129,388, 7,073,415, 7,238,204 and 7,166,108, each of which is fully incorporated by reference herein. Spinal fixation systems generally inhibit movement in the affected regions post-implantation.

In some procedures, spinal fixation provides rigid support (i.e., in a fusion procedure) for the affected regions of the spine to severely restrict/limit movement of the affected regions of the spine in virtually all directions. More recently so-called "dynamic" stabilization systems have been introduced wherein the spinal implant allows at least some movement of the affected regions in at least some direction (i.e., flexion, extension, lateral, or torsional movement). In certain circumstances, a dynamic stabilization system can benefit from the use of a stabilization rod that provides some movement in certain directions, while still providing support and stabilization to the overall stabilization system.

### SUMMARY

Embodiments disclosed herein can be used to stabilize parts of the human body, and in particular, are described in conjunction with providing stabilization of the spine The various embodiments disclosed herein will describe a spinal stabilization rod that can provide dynamic support and/or stabilization of a portion of the spine. As one skilled in the art can appreciate, embodiments of stabilization rods disclosed herein are not limited to dynamic stabilization of the spine and can be applied to and adapted for other surgical applications where providing stabilization or support with some movement is desired.

In one embodiment, a connection member includes a first rigid section, a second rigid section, and a flexible section joined between the first and second rigid sections. In one aspect of this embodiment, the flexible section can be joined to the first and second rigid sections by molding a material to contact connecting portions of each of the first and rigid sections, where the material retains some flexibility relative to the rigid sections post-molding. In some embodiments, the first and second rigid sections are made from a metal, for example titanium, titanium alloys, stainless steel and steel alloys or other suitable rigid materials as would be understood by one of ordinary skill in the art. In some embodiments, the flexible section comprises material with flexibility relative to the rigid sections, for example a moldable material such as polycarbonate urethane or other materials as would be understood by one of ordinary skill in the art.

In another embodiment, the connecting portions of the first and second rigid sections can have an exterior surface where all or a portion of the exterior surface has been adapted to have an increased surface area so as to inhibit separation of the flexible section from the first and second rigid sections. In some embodiments, this increased surface area can constitute an irregular surface made up of a plurality of randomly sized, as defined by height and thickness, and spaced, as defined by proximity there between, protrusions which are substantially unstressed with respect to the exterior surface and which are constructed monolithically therefrom. The connecting portion of the first and second rigid sections can also include one or more features that can further aid in resisting separation of the flexible section from the first and/or second rigid sections. These features can extend radially outward or recess radially inward from the exterior surface of the connecting portions of the first and second rigid sections. In some embodiments, these increased surface area features can include among other features an aperture, an undercut, a protrusion and/or a boss.

In another embodiment, a contact point between the molded material of the flexible section and the first and second rigid sections acts as a barrier against ingress of contaminants between the first/second rigid sections and the flexible section. In some embodiments, the junction between the molded material of the flexible section and the connecting portion of the first rigid section forms this contact point.

In another embodiment, a method of forming a spine stabilization rod is disclosed. In one embodiment, forming a connection member may include forming a first rigid section with a connecting portion having an exterior surface, forming a second rigid section with a connecting portion having an exterior surface, wherein at least a portion of these exterior surfaces are have an increased surface area, and molding a material about the connecting portion of the first rigid section and the connecting portion of the second rigid section to form a flexible section. In one embodiment, the method can include forming an irregular surface on the exterior of the connecting portions of the first and/or second rigid section. In one embodiment, forming the irregular exterior surface can be accomplished as follows: masking the exterior surface in a random pattern with a maskant material so that less than the entire surface portion is covered thereby, etching (for example electrochemically) the surface portion such that material from the first and second rigid section is removed in areas uncovered by the maskant material and areas covered by the maskant material are left intact, removing the maskant material, and repeating the masking, etching and removing steps upon the surface portion until a desired surface irregularity is achieved.

In another embodiment, a spine stabilization system can include a connection member as described above, and two or more anchoring systems for affixing the connection member to two or more vertebrae.

Other features, advantages, and objects of the disclosure will be better appreciated and understood when considered in conjunction with the following description and the accompanying drawings.

### BRIEF DESCRIPTION OF THE DRAWINGS

A more complete understanding of the present disclosure and the advantages thereof may be acquired by referring to the following description, taken in conjunction with the accompanying drawings in which like reference numbers indicate like features and wherein:

FIGURE 1 depicts a simplified graphical representation of a side view showing a portion of a healthy spine;

FIGURE 2 depicts a simplified graphical representation of a side view showing a portion of a weakened/damaged spine;

FIGURE 3 depicts a simplified graphical representation of a side view showing a portion of a healthy spine and various types of movements of the spine;

FIGURE 4 depicts a schematic representation of a spine in various positions to demonstrate various potential movements;

FIGURE 5 depicts a perspective view of one embodiment of a connection member;

FIGURE 6 depicts a perspective view of one embodiment of a rigid section of a connection member;

FIGURE 7 depicts a perspective view of another embodiment of a rigid section of a connection member;

FIGURE 8 depicts a perspective view of another embodiment of a rigid section of a connection member;

FIGURES 9A-9C depict partial views of various embodiments of the exterior surface of the connecting portions of rigid sections formed to have an irregular surface;

FIGURE 10 depicts a partial perspective view of one embodiment of a flexible section of a connection member with rigid sections as shown in FIGURE 8;

FIGURE 11 depicts a cross-sectional view the embodiment of a connection member of FIGURE 10;

FIGURE 12 depicts a cross-sectional view of another embodiment of a connection member;

FIGURE 13 depicts a cross-sectional view of another embodiment of a connection member;

FIGURE 14 depicts a perspective view of an alternative embodiment of a connection member; and

FIGURE 15 depicts a simplified posterior view showing a spinal stabilization system installed on vertebral bodies and having a pair of connection members in accordance with embodiments disclosed herein.

### DETAILED DESCRIPTION

The disclosure and the various features and advantageous details thereof are explained more fully with reference to the non-limiting embodiments detailed in the following description. Descriptions of well known starting materials, manufacturing techniques, components and equipment are omitted so as not to unnecessarily obscure the disclosure in detail. Skilled artisans should understand, however, that the detailed description and the specific examples, while disclosing preferred embodiments of the disclosure, are given by way of illustration only and not by way of limitation. Various substitutions, modifications, and additions within the scope of the underlying inventive concept(s) will become apparent to those skilled in the art after reading this disclosure. Skilled artisans can also appreciate that the drawings disclosed herein are not necessarily drawn to scale.

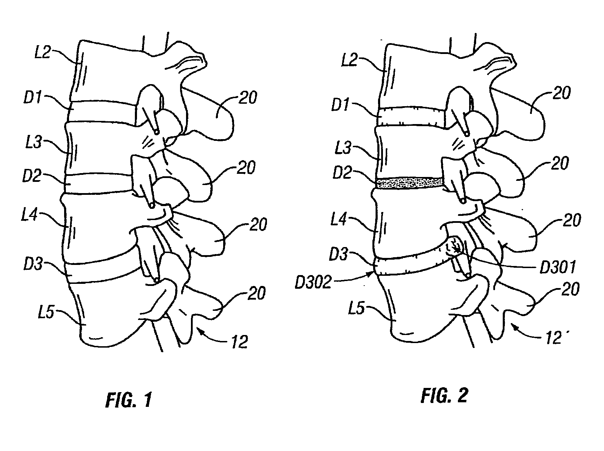

FIGURE 1 depicts a simplified graphical representation of a side view of a portion of a healthy spine, which is composed of vertebral bones (i.e., vertebrae) stacked up on one another in a smooth alignment. The spinal canal sits within the spinal column and houses the spinal cord and spinal nerves that send signals to and from the brain. The linkages between the vertebrae are soft, with discs in the front and ligaments in the back, allowing displacement and adaptation to stress and load. Due to the unique and complex arrangements and configurations of vertebrae (e.g., vertebral bodies 20 and vertebral segments L2, L3, L4, and L5) and intervertebral disc components, a healthy spine (e.g., spine 12) is strong and flexible and can function through the vigorous demands of daily living, work, and recreational activities. However, the spine is vulnerable to injury and to degeneration, a term which refers to the gradual failure of the spine's biomechanical functions due to aging and wear and tear. The soft tissues (e.g., the intervertebral discs, the ligaments and cartilage of the facet joints) are most vulnerable to degeneration. With normal aging, the discs would gradually collapse and ligaments lose their elasticity and stabilizing ability. Other factors such as those described below may also cause damage to the spine.

FIGURE 2 depicts a simplified graphical representation of a side view of a portion of a weakened/damaged spine (e.g., spine 12'). The cause of the damage may be physical (e.g., trauma), natural (e.g., aging/degeneration), disease (e.g., cancer) or a combination thereof. To provide examples, all three discs (D1, D2, and D3) shown in FIGURE 2 have abnormalities. Specifically, D1 has a slightly reduced disc height with minimal deterioration, D2 is severely damaged by the degenerative disc disease (DDD), and D3 is a herniated disc with D302 pointing to a bulging part of D3 and D301 pointing to the herniation of D3 where a nerve root of the spinal cord is pinched. In the case of DDD, the central nucleus dehydrates and loses its ability to transfer loads to annulus. As is known, disc degeneration leads to disc height loss, altering the normal spinal biomechanics and motion.

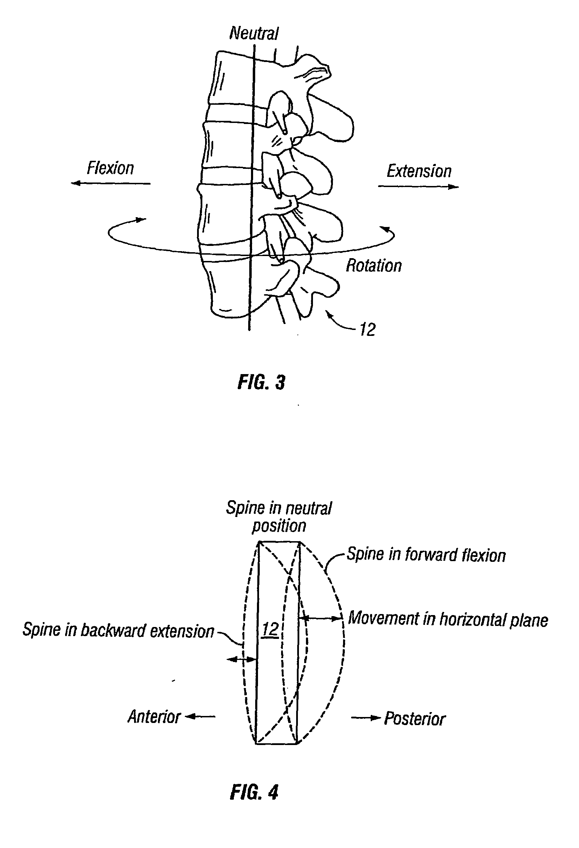

FIGURE 3 depicts a simplified graphical representation of a side view of a portion of healthy spine 12 and various types of movements thereof. FIGURE 4 depicts a schematic representation of spine 12 in various positions corresponding to the flexion-extension range of motion of the spine in the horizontal plane.

Surgical approaches used to stabilize the spine include spinal fusion and non-fusion treatment options. Spinal fusion, in simplest terms, is a surgical method for removing the damaged intervertebral disc and growing bone structures together to create a solid bone bridge between vertebrae. A fusion of the spine can be done by way of various known methods. The ideal technique in a particular patient will depend upon a number of factors including, but not limited to, the level(s) of vertebrae to be fused, degree of instability or deformity of the spine, age of the patient, risk factors for non-union (i.e., failure to fuse properly), and experience of the surgeon. Known spinal fusions include anterior spinal fusion, posterior spinal fusion with spinal instrumentation, posterior spinal fusion without spinal instrumentation, circumferential fusion (anterior and posterior), posterior lumbar interbody fusion, and transforaminal lumbar interbody fusion. The last two spinal fusion techniques are less invasive than the circumferential fusion and may decrease complications related to open exposures (e.g., infection, wound healing problems, etc.).

One method of stabilizing a portion of the spine may include coupling a connection member to the spine. Various embodiments are disclosed for a connection member that can include at least one flexible section coupled between two rigid sections, where the flexible section is flexible relative to the rigid sections. In one embodiment, a connection member may be a spine stabilization rod. In a spine stabilization system, the rigid sections of the spine stabilization rod may be coupled to an anchoring device, such as a bone fastener, and, in some embodiments, the flexible section can be aligned with a damaged disk or other portion of the spine that needs stabilization. The flexible section could have varying degrees of flexibility with respect to the rigid sections.

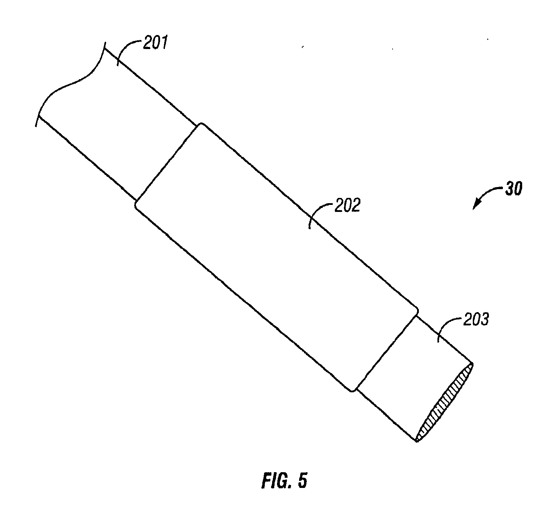

FIGURE 5 depicts a perspective view of one embodiment of connection member 30, for example a spinal stabilization rod, having a first rigid section 201, a second rigid section 203 and flexible section 202 joined between them. As shown in the FIGURE 5 embodiment, flexible section 202 has a larger outer diameter than the outer diameter of rigid section 201 or 203. In alternative embodiments flexible section 202 may have a smaller outer diameter than the outer diameter of rigid sections 201 or 203. In some embodiments, a portion of flexible section 202 may overlap a portion of first rigid section 201 and/or second rigid section 203. While the embodiment of connection member 30 of FIGURE 5 has a substantially circular longitudinal cross-section, in alternative embodiments, connection members 30 may have other cross-sectional shapes including, but not limited to, regular shapes (oval, rectangular, rhomboidal, square) and irregular shapes.

The materials used for rigid sections 201 and 203 and the material used for flexible section 202 may be selected so that a significant amount of flexion or extension is allowed by connection member 30 and/or flexible section 202. In some embodiments, the amount of flexion provided by connection member 30 or flexible section 202 may be approximately 0.1 to 1.0mm, and in a particular embodiment approximately 0.5 mm, while the amount of extension provided by connection member 30 (or flexible section 202) may be approximately 0.5mm to 1.5mm, and in a particular embodiment approximately 1.0 mm. It should be understood that the amount of flexion or extension provided by any particular connection member 30 of the present embodiment can be higher or lower than these numbers, and can be adjusted according to the particular dynamic stabilization need.

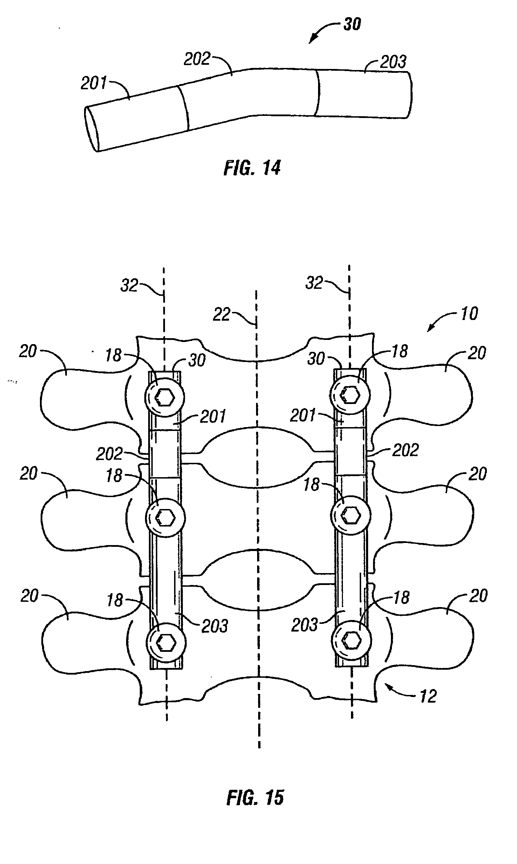

In some embodiments, rigid sections 201 and 203 may be made of any biocompatible materials capable of providing a desired rigidity and/or desired modulus of elasticity. In some embodiments, rigid sections 201 and 203 may be manufactured from titanium or a titanium alloy, stainless steel or an alloy, some combination thereof, or some other biocompatible metal. In some embodiments, rigid sections 201 and 203 may be manufactured having a durometer of 55 Shore D or greater. In alternative embodiments, first rigid section 201 and/or second rigid section 203 may be formed from a flexible polymer having a flexibility less than that of flexible section 202. Examples of polymers might include polyethylethylketone (PEEK), polyethylketoneketone (PEKK), and PMMA. Rigid sections 201 and 203 may be of the same or different lengths relative to one another and relative to flexible section 202. For example, in some embodiments, first rigid section 201 may be long enough to span between three vertebrae and second rigid section 203 may be long enough to span between only two vertebrae. In some embodiments, rigid sections 201 and/or 203 may be of a length that allows connection member 30 to be coupled to anchor system 18 (see FIGURE 15).

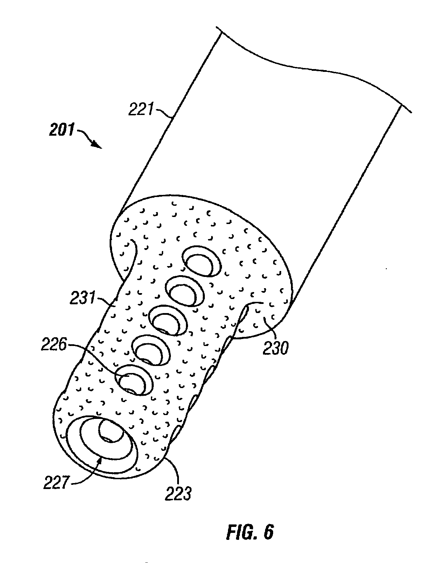

FIGURES 6, 7 and 8 depict perspective views of various embodiments of rigid sections 201 and/or 203 of connection member 30. With reference to FIGURE 6, rigid sections 201 and 203 include first portion 221 and connecting portion 223, each of which may be formed by machining, molding, or other suitable manufacturing techniques. In some embodiments, first portion 221 of rigid section 201 or 203 may be formed to allow for coupling with other components of spine stabilization system 10 (for example, having an outer dimension suited for coupling with an appropriate anchoring system). For example, first portion 221 may have a generally circular cross-sectional shape with a constant outer diameter (i.e., a cylindrical shape) such that first portion 221 is suited for coupling with a collar. In some embodiments, connecting portion 223 may be formed having a smaller diameter than first portion 221, such that surface 230 is formed having an associated surface width.

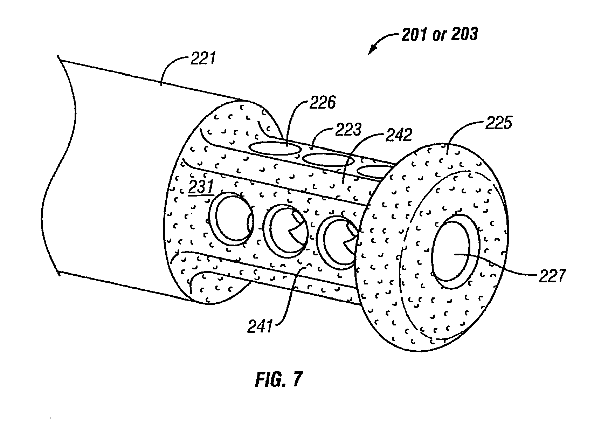

As shown in the embodiment of FIGURE 6, connecting portion 223 may be formed to have a substantially constant cross-sectional profile. To manufacture connecting portions 221 of FIGURE 6, rigid sections 201 or 203 can be made of a metal that can be turned or otherwise formed to have a continuous exterior surface 230/231 prior to any further surface processing. In an alternative embodiment, FIGURE 7 depicts rigid sections 201 and/or 203 in which connecting portion 223 may have a plurality of sides 241 and 242 such that connecting portion 223 can has a constant, but non-circular cross-sectional shape. A constant cross-sectional profile of rigid sections 201 and 203 can provide, in some applications, a more uniform stabilization capability in a variety of movement directions.

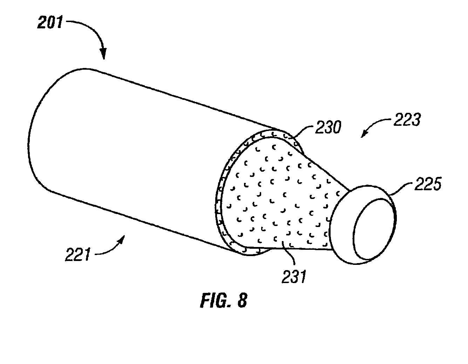

In alternative embodiments, the cross-sectional profile of connecting portion 223 may increase or decrease along the longitudinal axis of connecting portion 223. While FIGURE 6 depicts rigid section 201 or 203 with connecting portion 223 having a generally constant outer diameter, FIGURE 7 shows rigid sections 201 or 203 with connecting portion 223 having a varying outer diameter, and FIGURE 8 shows an embodiment of rigid section 201 or 203 having connecting portion 223 with a generally decreasing outer diameter or tapered profile.

In various embodiments, connecting portion 223 may be formed to have features extending radially outward or recessing radially inward from an outer surface. These features can include, for example, an aperture, an undercut, a protrusion and/or a boss. These features can increase the surface area for a given length of connecting portions 223, which may improve the connection between rigid section 201 or 203 and flexible section 202. In other words, as compared to a rigid section having a constant diameter and a smooth, continuous surface, a taper, bevel, boss, undercut, aperture, cannula or some other discrete or continuous feature can be used to increase the surface area of connecting portion 223 for a given length of connecting portion 223. For example, as shown in the embodiment of FIGURE 6, connecting portion 223 of rigid section 201 (or 203) may include apertures 226 and cannula 227. During the manufacturing process, in certain embodiments the flexible material that forms flexible section 202 can enter apertures 226, cannula 227 or both. For example, if flexible section 202 is formed by molding a flexible material around connecting portions 223, during the molding process the flexible material can enter the apertures 226 and cannula 227 while in an uncured state and harden during the molding process. The presence of the flexible material in apertures 226 and/or cannula 227 can inhibit separation of rigid sections 201 and 203 from flexible section 202, and may provide additional support, such as by inhibiting torsional strain.

The embodiments shown in FIGURES 6 and 7 show connecting portion 223 having apertures 226 recessing into connecting portion 223 for potentially receiving flexible material of flexible section 202. In certain, these apertures 226 in connecting portion 223 can be positioned on one or more axes or on one or more sides. Apertures 226 may recess completely through or to a limited depth through connecting portion 223. FIGURE 6 shows an embodiment of rigid section 201 (or 203) having sets of five apertures 226 recessing into connecting portion 223 along two axes. FIGURE 7 depicts an alternative embodiment having sets of three apertures 226 along two axes. Those skilled in the art will appreciate that the number, size and placement of apertures 226 can be altered to provide a desired connectivity between flexible section 202 and rigid section 201 or 203. FIGURES 6 and 7 further depict embodiments of connecting portion 223 having cannula 227 recessing along a portion of the length thereof, where the apertures 226 are recessed through connecting portion 223 and intersect cannula 227. Once again, cannula 227 can also receive flexible material of flexible section 202 during the manufacturing process (in one example, during injection molding to form flexible section 202).

FIGURE 7 depicts an embodiment in which connecting portion 223 has a combination of features including sides 241, sides 242, apertures 226, cannula 227 and boss 225, where boss 225 radially extending from connecting portion 223. In some embodiments, forming exterior surface 231 of connecting portion 223 involves turning connecting portion 223 to a desired diameter, then facing or otherwise machining sides 241 and sides 242, whereby sides 241 are flat and sides 242 are radiused. In some embodiments, forming surface 231 may include turning connecting portion 223 to a desired diameter, then facing or otherwise machining sides 241 and facing or otherwise machining sides 242, whereby sides 241 and sides 242 are flat. Forming connecting portion 223 can further include removing material via machining techniques to form apertures 226 and cannula 27. Those skilled in the art will appreciate that sides 241 and 242 may be formed on exterior surface 231, or a combination thereof to provide a complex profile on connecting portion 223. A potential advantage to having multiple sides 241 and/or 242 may be the increase in torsional strength of connection member 30. In some embodiments, flexible material may be molded around conneding portion 223 such that material enters apertures 226 or cannula 227, envelops boss 226, or adheres to one or more of sides 241 and 242 to create flexible section 202 between first rigid section 201 and second rigid section 203. The various features of sides 241 and 242, apertures 226, boss 225, and/or cannula 227 can potentially increase the ability to withstand shear stresses from tension, extension, bending or torsion of the spine and also inhibit the separation of flexible section 201 from rigid sections 201 and 203.

FIGURE 8 shown another embodiment of rigid section 201 or 203 where connecting portion 223 has a tapered profile (where the outer diameter of connecting portion 223 is greater proximate first portion 221 and is smaller distal first portion 221) and boss 225 extending radially therefrom.

In some embodiments, once one or more desired features have been formed on connecting portion 223, connecting portion 223 may be exposed to chemical and/or mechanical processes to make the exterior surface 231 (and/or 230) of connecting portion 223 irregular. As illustrated in the embodiments of FIGURES 6, 7 and 8, exterior surface 231 (and/or 230) of connecting portion 223 can be irregular. An irregular exterior surface 231 (and/or exterior surface 230) can provide an increased surface area and further inhibit separation between flexible section 202 and first rigid section 201 and/or second rigid section 203. Connecting portion 223 may be chemically/mechanically etched and/or machined to form a desired irregular exterior surface 231 or 230.

Increasing the exterior surface area of connecting portion 223 may include forming a desired surface pattern on connecting portion 223 by removing material from the exterior surface 231 or 230 of connecting portion 223. Etching is one method of removing material to provide an irregular surface. In some embodiments, etching may include the steps of masking a surface portion in a random pattern with a maskant material, such that less than the entire surface portion is covered thereby, electrochemically etching the surface portion such that first rigid section material or second rigid section material is removed thereby in areas uncovered by the maskant material and areas covered by the maskant material are left intact, removing the maskant material, and repeating the masking, electrochemically etching and removing steps upon the surface portion until a desired surface irregularity is achieved.

In one embodiment, chemical etching can be employed to create an irregular exterior surface for the connecting portion 223 of rigid section 201 and/or 203. For purposes of describing the chemical etching, this description will refer to the use of a rigid section made from a titanium alloy base metal. While titanium represents one embodiment, the rigid section may be formed from a number of other alloys may be utilized. Each of these different alloys will require a different maskant and etchant composition. It is, however, considered to be within the knowledge of a person of ordinary skill in the art to select an appropriate etchant based on the base alloy selected. Furthermore, for the purposes of clarity, certain repetitive elements in the drawings have not been numerically identified for each and every occurrence. For example, a number of maskant points are shown on the surface diagrams. It is considered apparent from the drawings that the maskant points and other surface features of the etched implant are repeated and are readily identifiable without the aid of numeric identification for each feature. Only representative features and maskant points have therefore been identified.

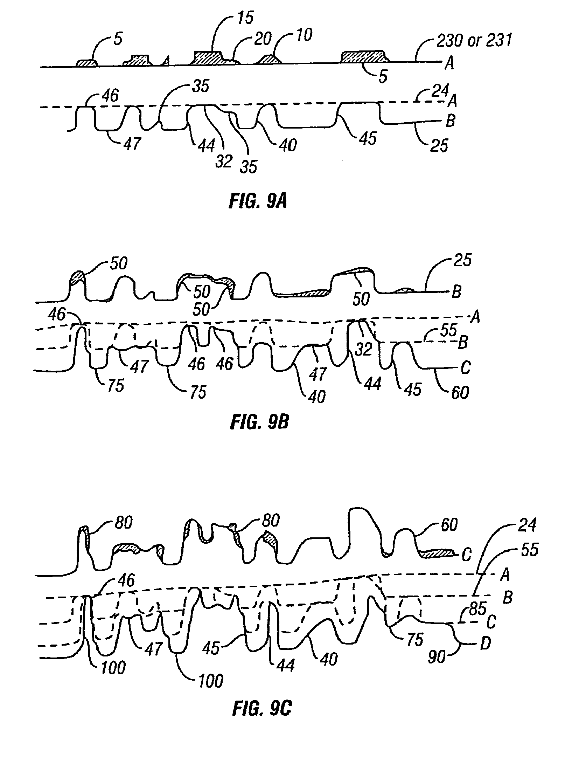

Referring now to FIGURES 9A-9C, various embodiments of an irregular exterior surface 231 of connecting portion 223 are shown in various stages of manufacture using chemical etching. In each of FIGURE 9A-9C, there are two illustrations where the upper illustration shows the exterior surface 231 prior to etching and the lower illustration shows exterior surface 231 post-etching. In FIGURE 9A, unfinished surface 231 (applicable also to surface 230) is shown which diagrammatically represents the exterior surface 231 of connecting portion 223. The letter identifiers on the right margin of the drawings are intended to provide a quick reference to the relative levels of etching. Unfinished exterior surface 231 at level A is generally smooth (e.g., post machining) and can be a titanium metal or alloy such as Ti-6AI-4Va. A maskant is applied to surface 230 or 231 of connecting portion 223 in order to perform a random etch. Several methods may be utilized to accomplish the random spattering of the maskant on surface 231. Among these are manually applying the maskant by brushing it using the tips of a hair-type brush or utilizing any type of shredded hair-like fibrous applicator dipped in the maskant material. Another method of application would be delivered in an air stream utilizing an air brush or paint gun.

The maskant must be chosen in order to provide a substance which will cling tightly to surface 231 of connecting portion 223 during manipulation of connecting portion 223 and will also remain stable when the etchant solution is applied to the coated portion. The maskant must also be removed with no residue once its function has been accomplished. A particular problem encountered when utilizing maskants is the performance of the maskant at the boundaries of its application. The maskant should produce a sharply defined edge once the etching process has begun and not itself deteriorate during the etching process. This might permit partial degradation of the substrate in a masked area. It should be noted, however, that some deterioration is found in any maskant use and does provide some of the particular surface features of the etched connecting portion 223 described later.

Exterior surface 231 of connecting portion 223 should be relatively clean and grease-free, with all or substantially all of any oxidized material removed before the application of the maskant. This may be accomplished either mechanically, chemically or both. Exterior surface 231 may be cleaned mechanically utilizing a light abrasive blast of aluminum oxide particles or glass beads. Alternatively, blasting with any small solid particle which will not degrade surface 231 is contemplated. A chemical agent such as methanol may be utilized alone or in conjunction with the blasting. Most maskants are sensitive to the condition of the applied surface and both application and removal of the maskant may be affected by improper surface treatment. The maskant can be comprised of a number of materials including neoprene elastomers and isobutylene isoprene copolymers. The particular maskant should be selected based on the type of etchant utilized. For the embodiment described, the maskant is AC-818C, an air-cured, general purpose, peelable coating produced by A.C. Products, Inc. of Placentia, Calif. The maskant is thinned utilizing perchlorethylene to 35-45 seconds utilizing a No. 5 Zahn cup. The maskant, if too thin, may be thickened to this viscosity by evaporation of the carrier. While the maskant is traditionally utilized in the 14-18 second range, it has been found that a thicker version can produce superior results in terms of applying the maskant utilizing manual daubing or spray application techniques. It is to be specifically noted that the maskant is applied in a random spattered fashion allowing only a portion of exterior surface 231 of connecting portion 223 to be coated thereby. A random "polka dot" pattern can be used in which each of the maskant points is of varying size and thickness when compared to the others. In some instances, the applied maskant may be partially abraded utilizing the grit blasting technique described previously for cleaning with an 80-120 mesh grit at 80-90 psi. to assist in providing an irregular maskant coating.

As shown in FIGURE 9A, a variety of applied maskant points 5 are illustrated. A relatively thick maskant agglomeration 10, an applied maskant plateau 15 and a relatively thin maskant layer 20 are all shown in FIGURE 9A. It is desirable to achieve a variety of sizes and thicknesses of maskant in order to obtain a random finished surface. As will be seen later, each of these particular maskant surface features produces a somewhat different etched result. An optional step of drying the maskant at an elevated temperature is also contemplated (for example four to five minutes at 200 degrees F).

Referring now to the illustration of FIGURE 9A, the resulting etched surface 25 is illustrated in the lower illustration, based on the etching given the applied maskant shown in the upper illustration of FIGURE 9A. The unfinished surface indication line 24, shown as a dotted line, indicates the original level (identified by the letter A) at which the surface 231 began. The first etched surface 25 represents exterior surface 231 of connecting portion 223 after the first etch (also indicated by the letter B).

While a number of etchants could be utilized, the particular chemistry adopted for the described embodiment utilizes a standard 30% nitric acid-6% hydrofluoric acid combination which is commonly marketed and well known in the art. The etchant is applied at 110 degrees F for approximately four minutes to achieve a desired 0.008-0.010 inch etch depth. This time period or the strength of the etchant solution may be adjusted upwardly or downwardly to achieve a heavier or lighter etching. The etching is halted in a water bath or spray. Post-etch the maskant material may be removed in a variety of ways, including mechanically or chemically. Depending on the size and number of coated objects, mechanical brushing or blasting of the maskant will peel it off. Additionally, the use of nitric acid is also contemplated to dissolve the maskant material.

Referring again to the lower illustration of FIGURE 9A, a number of surface features may be identified. A primary plateau 32 corresponds to the applied maskant plateau 15 illustrated in the top drawing. The heavy maskant coat 15 nearly or completely protected surface 231 at that point, thereby preventing metallic material from being removed at this point. A secondary plateau 35 corresponds to the thin layer 20 illustrated in the top drawing of FIGURE 9A. The intermediate height of the secondary plateau between levels A and B indicates that the maskant performed for some period during the etching cycle but failed at an intermediate time allowing some of the alloy to be etched away. Item 35 of the lower illustration of FIGURE 9A illustrates a small secondary plateau. Gradually sloped feature 40 corresponds to a gradually tapering maskant coverage which partially protected the underlying substrate during the etching cycle. Highly sloped feature 44 indicates a thicker maskant coating which had a highly defined perimeter before etching. Medium sloped feature 45 indicates a maskant condition intermediate to the two previously described. The extremes of the etching are indicated by unetched level 46 and first etched level 47 which illustrate the effect of complete maskant coating versus no maskant coating. It should be noted that the base of each surface feature provides full dimensionally filleted radii.

FIGURE 9B again employs two illustrations to display the effects of a second masking/etching cycle. The upper illustration shows irregular exterior surface 25 of FIGURE 9A (corresponding to exterior surface 231 post the first etch cycle) where the lowest extreme is shown at the level indicated by the letter B. The lower illustration of FIGURE 9B shows irregular exterior surface 60 corresponding to exterior surface 231 after the second etch cycle (where the lowest extreme is now shown at the level indicated by the letter C). Maskant is again applied to a clean and prepared exterior surface 231 in a random fashion according to the same techniques described above and as generally indicated in the upper illustration of FIGURE 9B. As before, a randomized pattern is preferable in which a wide variety of maskant surface features is achieved. Second applied maskant points 50 illustrate a variety of positions in which the maskant may be applied to the now irregular surface features of first etched surface 25.

As shown in the lower illustration of FIGURE 9B, the first etched surface 25 is shown as a dotted indication line 55 (to indicate the previous surface 25 prior to the second etching cycle). The second etching cycle is performed under the same or very similar conditions as that described with reference to FIGURE 9A to again achieve a 0.008-0.010 inch maximum etch. Second etched surface 60 is shown at level C, indicating a resultant etched exterior surface 231 after the second etch cycle. As previous described, the number of surface features are illustrated corresponding to the characteristics of the applied maskant. A highly sloped surface feature 44 corresponds again to a sharply defined and relatively thick application of maskant while a gradually sloped surface feature 40 corresponds to a gradually thinning maskant application. This feature is particularly visible in the two illustrations contained in FIGURE 9B in which the gradual thinning of the maskant application is particularly exaggerated.

As can be seen in the lower illustration of FIGURE 9B, three major levels of surface features are illustrated with a few intermediate features present to demonstrate the effects of partial maskant failure. A few points remain at unetched level 46 indicating maskant coverage during both etchant cycles. Some points are illustrated at first etched level 47 indicating maskant coverage during one of the two cycles, while points located at second etched level 75 have been exposed to the etchant during both of the etching cycles. The increasing level of complexity of surface forms is apparent by comparing the lower illustration of FIGURE 9A (showing exterior surface 231 after the first etch cycle) with the lower illustration of FIGURE 9B (showing exterior surface 231 after the second etch cycle).

FIGURE 9C once again employs two illustrations to display the effects of a third masking/etching cycle. The upper illustration shows irregular exterior surface 60 of FIGURE 9A (corresponding to irregular exterior surface 231 post the second etch cycle) where the lowest extreme is shown at level C. The upper illustration of FIGURE 9C shows the application of third applied maskant points 80 to the now highly featured second etched surface 60. In the lower illustration of FIGURE 9C, dotted line 24 shows exterior surface 231 prior to the first etching, dotted line 55 shows exterior surface 231 after the first etching, dotted line 85 shows exterior surface 231 after the second etching and full line 90 shows exterior surface 231 after the third etching cycle. The increasing complexity of the exterior surface 231 of the etched connecting portion 223 can contributes to the complexity of the maskant forms when applied to the irregular exterior surface 231. The lower illustration of FIGURE 9C demonstrates the effect of a less rigorous etching cycle, being roughly one-half of the depth shown in FIGURES 9A and 9B. The number and length of each etching cycle is purely dependent on the complexity of features required by the application and may be performed by any order. As shown in the lower illustration of FIGURE 9C, a gradually sloped surface feature 40 retains its gradually sloped character from one cycle to the next when not covered by a maskant. This is to illustrate the consistent and uniform attack on the surface by the etchant solution. Highly sloped surface feature 44 again illustrates the effect of a highly stable maskant agglomeration while medium sloped surface feature 45 again demonstrates an intermediate condition. As illustrated in the lower illustration of FIGURE 9C, four major surface levels (indicated by letters A, B, C and D) are shown. Points at unetched level 46 are still apparent although fewer in number and relatively rare. A number of plateaus remain at first etched level 47 and second etched level 75. Those areas which have been exposed during all three etchant cycles enjoy depressions at third etched surface 100 corresponding to level D in FIGURE 9C. These levels correspond to areas which have had coverage during all three cycles, two cycles, one cycle and no cycles, respectively. The result as shown by third etched surface 90 is of a highly non-uniform featured surface which, compared with its length, also exhibits a large surface area. The different levels of depression and protrusion are particularly adapted to permit the flexible material of flexible section 202 to join with in order to inhibit separation of flexible material 202 from first rigid section 201 and/or second rigid section 203.

After performing the process described above in conjunction with FIGURES 9A-9C, the connecting portions 223 of the first and second rigid sections 201 and 203 can have an irregular exterior surface 231 that provides an increased surface area that can inhibit separation of the flexible section 202 from the first rigid section 201 and second rigid section 203. In some embodiments, particularly when using the etching methods described herein, this irregular exterior surface 231 will be made up of a plurality of randomly sized, as defined by height and thickness, and spaced, as defined by proximity there between, protrusions which are substantially unstressed with respect to the exterior surface and which have been constructed monolithically therefrom.

In addition to chemical etching as described above, the irregular exterior surface 231 (or 230) of connecting portion 223 can also be achieved using electrochemical etching. In one embodiment, electrochemical etching can be performed on the connecting portion 223 of rigid sections 201/203 made from a cobalt-chromium alloy base metal. While cobalt-chromium alloy is the described embodiment, a number of other alloys may be utilized in connection with electrochemical etching. Each of these different alloys may require a different maskant and electrochemical etching conditions. While no specific details are given in the specification regarding the use of these other metals and electrochemical etching conditions, it is considered to be well within the knowledge of a person of ordinary skill in the art to select the appropriate electrochemical etching conditions once a base alloy has been identified.

Once again referring now to FIGURES 9A-9C, various embodiments of an irregular exterior surface 231 of connecting portion 223 shown in various stages of manufacture can be accomplished using electrochemical etching. The maskant is once again applied to the exterior surface 231 which is to be electrochemically etched in a random fashion, as previously described. The maskant described herein for use with cobalt-chromium alloys is an alkaline soluble, air-curable phenol-formaldehyde resin maskant material such as Hysol ER1006 produced by The Dexter Corporation, Industry, Calif. For the Hysol ER1006 maskant, a useful viscosity is about 60-66 seconds as measured utilizing a No. 5 Zahn cup. After the maskant has been applied in a random spattered pattern, it is cured. For example, the Hysol ER1006 maskant is preferably cured for a minimum of about 20 minutes at between about 200-250 degrees F and then air cooled to room temperature.

As described previously, the maskant is applied at a variety of points in a variety of stages. A typical electrochemical etching process involves applying a maskant material and curing the applied maskant material until the exterior surface 231 of connecting portion 223 is ready to be electrochemically etched. A tank may be used to submerge the tooling and connecting portion 223 under an electrolyte fluid. The electrolyte fluid fills the work gap between the tooling and the exterior surface 231 of connecting portion 223. The electrolyte fluid is pumped at controlled rate through a passageway in the tooling and out through an orifice into the work gap. The tooling is in electrical connection with the negative terminal of a direct current power supply and thus becomes the cathode of the electrochemical etching process. Connecting portion 223 is in electrical connection with the positive terminal of the same direct current power supply and thus becomes the anode of the electrochemical etching process.

The electrolyte fluid for electrochemically etching a cobalt-chromium alloy is preferably a solution containing the proportions of one pound each of NaCl and NaNO₃ dissolved in one gallon of water. One skilled in the art of electrochemically etching metals will recognize and employ the appropriate electrolyte fluid to be used for the type of metal of a particular connecting portion 223. Control of the flow rate of the electrolyte fluid through the work gap is important because the electrolyte fluid must adequately remove both the heat and the reaction products of the electrochemical process. The optimum flow rate level is related to the amount of current employed. Higher ratios of flow rate to current give better removal of heat and reaction products. For the electrochemical etching a cobalt-chromium alloy, for example, the electrolyte fluid should flow through the work gap at a rate of about 0.15-0.5 gallons per minute per 100 amps and have a temperature of between about 100-130 degrees F. One skilled in the art of electrochemically etching metals will be able to determine the proper values of these parameters to use with a particular application.

The tooling may be made from any material suitable for use in electrochemical etching such as copper, nickel, or an alloy of tungsten-copper. The tooling should be configured so that the work gap between the tooling and the attachment surface is substantially uniform. This is accomplished by making the tooling substantially conformal to exterior surface 231. In one embodiment, the work gap is between approximately 0.020-0.250 inches, and in a more particular embodiment between approximately 0.060-0.120 inches. One skilled in the art of electrochemically etching metal will be able to determine the proper work gap to use for a particular application. A direct current voltage difference between the tooling and exterior surface 231 of between about 8V-24V and a specific amperage of at least about 50 amps per square inch of exposed portion 120 of exterior surface 231 are to be maintained during the electrochemical etching of a cobalt-chromium connecting portion 223. Preferably, the direct current voltage difference between the tooling and connecting portion 223 is between about 12-18V and the specific amperage is about 75-120 amps per square inch of exposed portion 120 of exterior surface 231. The values of these parameters for use with other materials are readily determinable by one skilled in the art of electrochemical etching metals. The stated conditions will produce a metal removal rate of about 0.003 inch per minute when the connecting portion 223 material is a cobalt-chromium alloy. The time period and other parameters of the electrochemical etching process, particular the specific amperage, may be adjusted upwardly or downwardly to achieve a heavier or lighter etching. The electrochemical etching process is halted by removing the voltage difference between the tooling and connecting portion 223.

The maskant material on exterior surface 231 is removed after each electrochemical etching step. In the embodiment using a cobalt-chromium alloy exterior surface 231 and the Hysol ER1006 maskant material, the exterior surface 231 can be immersed in an aqueous solution of an alkaline cleaner to dissolve the maskant material, where the temperature of the alkaline cleaner solution is between about 80-145 degrees F. The immersion time can be about 5 to 10 minutes or until the maskant is removed. Water blasting is employed to remove any clinging maskant material which was softened by the alkaline cleaning solution.

In the embodiment shown in FIGURES 9A-9C, the masking/electrochemical etching process is repeated three times (though it should be understood that useful irregular attachment surfaces may be obtained through the use of fewer and more numerous cycles). The amount of material removed during each cycle is to be determined by the particular application. In one embodiment, substantially the same amount of material, as measured by depth of material removal, is removed in each cycle. When multiple masking/electrochemical etching cycles are employed, exterior surface 231 can be blasted with 80 to 120 mesh alumina grit prior to the application of the maskant material so as to promote the adhesion of the maskant material. The resultant irregular exterior surface 231 of connecting portion 223 formed from electrochemical etching is once again illustrated by item 90 in FIGURE 9C.

Additional and/or alternative methods of forming an irregular surface on the exterior surface 231 of connecting portion 223 of first rigid section 201 and/or second rigid section 203 may be found in U.S. Patent Nos. 5,258,098, 5,507,815, 5,922,029 and 6,193,762, each of which is hereby fully incorporated by reference herein.

As described earlier, embodiments of connection member 30 can comprise flexible section 202 molded around connecting portion 223 of first rigid section 201 and connecting portion 223 of second rigid section 202. In one embodiment, first rigid section 201 and/or second rigid section 203 will have had an appropriate exterior surface 231 formed (to include an irregular surface area, one or more features or both) on at least a portion of the connecting portion 223, then rigid sections 201 and 203 may be placed in a mold (not shown) that can be used in a molding process (such as an injection molding process) to create flexible section 202 molded about connecting portions 223 of first and second rigid section 201 and 203. FIGURES 8, 10 and 11 depict views of embodiments of portions of connection member 30 at various stages of an exemplary molding process.

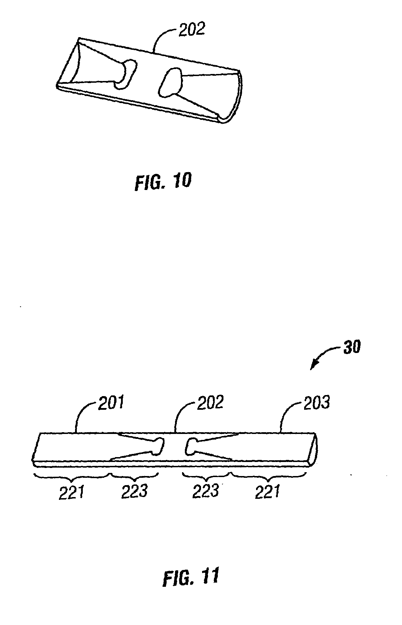

Once rigid sections 201 and 203 are positioned in the mold, material may be injection molded or otherwise molded around connecting portions 223 of rigid sections 201 and 203 to form flexible section 202 joined between the two rigid sections. FIGURE 10 depicts a cutaway view of a flexible portion 202 formed by molding flexible material around the connecting portion 223 of the embodiment of rigid section 201 shown in FIGURE 8. FIGURE 11 depicts a simplified perspective cutaway view of one embodiment of connection member 30 comprised of flexible section 202 molded to the embodiment of rigid sections 201 and 203 shown in FIGURE 8. As shown in the FIGURE 11 embodiment, flexible section 202 is formed having a cylindrical body extending along a longitudinal axis of connection member 30. It should be understood that various embodiments of rigid sections 201 and 203 can be joined to various embodiments of flexible section 202, as would be apparent to one of ordinary skill in the art.

In some embodiments, flexible section 202 may be made of a material with properties that mimic or more naturally follow a natural human body response to applied loading. In many embodiments, flexible section 202 can be formed from a material has a modulus of elasticity that is less than the modulus of elasticity of rigid sections 201 and 203. Examples of suitable materials for flexible section 202 include polycarbonate urethane (PCU), Polyetheretherketone (PEEK), Polyetherketoneketone (PEKK), Ultra high molecular weight polyethylene (UHMWPE) or other similar materials. Other suitable materials are possible and would be known to those skilled in the art. Thus, as an example, if rigid sections 201 and 203 are manufactured from a material with a high modulus of elasticity (e.g., titanium), a material such as PEEK might be useful for flexible section 202, but if rigid sections 201 and 203 are manufactured from PEEK, it is preferable that flexible section 202 be manufactured from a material more flexible than PEEK.

The flexible material of flexible section 202 may be molded around connecting portion 223 so as to adhere to exterior surfaces 230 and 231 of connecting portion 223. Adherence may include, for example, material penetrating into apertures 226 or cannula 227, or enveloping boss 225. Adherence may also include material penetrating into portions of irregular exterior surface 231 formed on connecting portion 231 (or 230) of rigid section 201 or 203. In some embodiments, material of flexible section 202 joined to exterior surface 230 can inhibit the ingress of microorganisms, moisture, or other contaminants into the interior of stabilization rod 20. In one embodiment, the adherence of material to surface 230 may withstand an autoclave process. An advantage to forming connection member 30 to withstand an autoclave process may be the ability to use non-sterile packaging to package/ship connection member 30 without risk of contamination. In some embodiments, the adherence of the material of flexible section 202 to exterior surface 230 can also improve resistance to shear forces applied to connection member 30 at the junction between flexible section 202 and rigid sections 201 or 203. Embodiments of connection member 30 having rigid sections 201 and 203 with irregular surface patterns on surface 230 in contact with the molded material used to form flexible section 202 may also be able to withstand greater shear forces to provide greater stability once connection member 30 is implanted (e.g., coupled to the spine). Those skilled in the art may appreciate, after reading this disclosure, that by adjusting the thickness t of surface 230, the resistance to contamination or the shear strength may be improved.

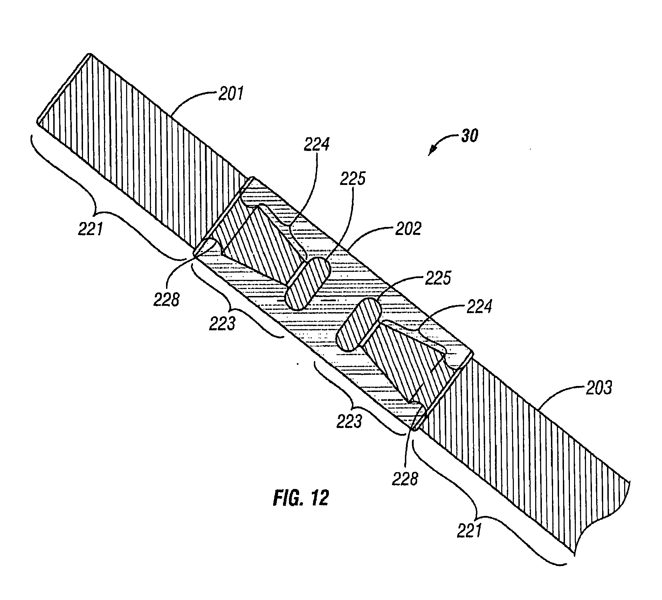

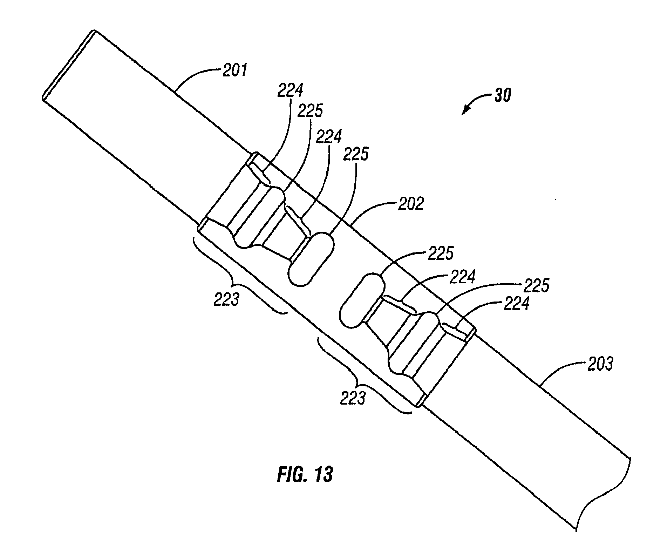

FIGURES 12 and 13 depict views of additional embodiments of connection member 30 having first rigid portion 201 connected to second rigid portion by flexible section 202. The first rigid section 201 and second rigid section 203 of FIGURES12 and 13 each have first portion 221 and connecting portion 223, where connecting portions 223 of FIGURE 12 have one tapered section 224 as compared to connecting portions 223 of FIGURE 13 that have two tapered sections 224 separated by intermediate boss 225. Each of FIGURES 12 and 13 is shown having boss 225 at the distal end of connecting potion 223. In both embodiments, each boss 225 radially extends some diameter less than the outer dimension of first portion 221, and may extend along the longitudinal axis of rigid section 201 or 203 some length. In some embodiments, the size, diameter, radius of curvature, length, or other characteristic of boss 225 may inhibit flexible section 202 from separating from rigid section 201 or 203, reduce the likelihood of fractures or other mechanical failure in flexible section 202, and/or improve the dynamic support of connection member 30 once connection member 30 is implanted. The FIGURE 12 embodiment of connection member 30 also includes an undercut 228 feature as part of connecting portion 223 to further increase the surface area of exterior surface 230/231 of connecting portion 223.

It should be understood that while embodiments of connection member 30 shown in the drawings herein have rigid sections 201 that are identical to second rigid sections 203, embodiments of connection member 30 may include first rigid section 201 having a surface area or geometry that is different from that of second rigid section 203. It should further be understood that while in some embodiments, rigid sections 201 and 203 are positioned in a mold such that their longitudinal axes are aligned, thereby resulting in a generally straight implantable connection member 30, in alternative embodiments, rigid sections 201 and 203 may be positioned in a mold such that their longitudinal axes are at some desired angle. For example, FIGURE 14 shows an embodiment of connection member 30 having flexible section 202 formed with an angle. In alternative embodiments, implantable connection member 30 may have a curve or bend formed in first rigid section 201, first rigid section 203, or some combination thereof.

In alternative embodiment, connection member 30 can comprise more than three sections, with each additional section being either a rigid section 201/203 or a flexible section 202. Depending upon the pattern and/or length desired, the biocompatible material used to form additional rigid sections or flexible sections may be the same or different from one another. In one embodiment, all parts of connection member 30 are of equal or substantially similar size. In one embodiment, the length of each part may be the same or substantially the same. In one embodiment, the length of each part may vary. In one embodiment, one or both ends (e.g., rigid sections 201 and/or 203) of connection member 30 can extend to more than one level, making connection member 30 useful in a multilevel procedure.

One of the advantages of various embodiments dynamic connection member 30 is that flexible sections 202 provide stability in lateral bending, axial rotation and in shear while allowing some motion that mimics the motion of a healthy spine. As one skilled in the art can appreciate, the geometry of connection member 30 can vary according to the need and the operating level (e.g., thoracic, lumbar, etc.).

FIGURE 15 depicts a simplified posterior view of one embodiment of stabilization system 10 for supporting spinal column 12 according to one aspect of this embodiment. Spinal stabilization system 10 may be installed posterior to spine 12 by coupling first rigid section 201 and second rigid section 203 of connection members 30 to vertebral bodies 20 using anchoring system 18 (for example using pedicle screws, hooks, clamps, wires, collars, rings, closure members, or other anchoring systems 18). As shown in FIGURE 15 stabilization system 10 comprises two connection members 30, each of which is affixed on either side of spine 12 along a longitudinal axis 32, parallel to the longitudinal axis 22 of the spine 12 lying in the mid-sagittal plane. However, one skilled in the art can appreciate that one or several connection members 30 may be utilized in various spinal stabilization systems.

Stabilizing a spine may include determining a desired rigidity for a first portion of the spine and a second rigidity for a second portion of the spine, and utilizing connection members 30having rigid sections 201 and 203 and flexible sections 202 of selected lengths to preserve the desired rigidity for different vertebral levels. For example, spine 12 may have anchoring systems 18 implanted in three or more vertebrae 20, such as depicted in FIGURE 15. Connection members 30 manufactured with rigid sections 201 and 203 and flexible sections 202 may be positioned relative to spine 12 and coupled to anchoring systems 18 such that rigid sections 203 span between vertebrae to promote fusion at selected vertebral levels and flexible sections 202 span between vertebrae to preserve motion at selected vertebral levels. For example, as depicted in FIGURE 15, connection members 30 may have rigid sections 203 connected to two anchoring systems 18 and having a length to rigidly stabilize a first vertebral level, rigid sections 201 anchored to a single anchoring system 18 and having a shorter length, and flexible sections 202 which are not coupled to anchoring systems 18 and having a length to span between rigid sections 201 and 203, such that one vertebral level is dynamically stabilized.

Spinal stabilization system 10 may also include suitable transverse rods or cross-link devices that help protect the supported portion of spine 12 against torsional forces or movement. Some possible examples of suitable cross-link devices are shown in co-pending U.S. Patent Application Serial No. 11/234,706, filed on November 23, 2005 and naming Robert J. Jones and Charles R. Forton as inventors (the contents of this application are hereby fully incorporated by reference herein). Other known cross-link devices or transverse rods may also be employed to provide further support to spinal stabilization system 10.

Spinal stabilization systems incorporating embodiments of connection member 30 can provide a number of advantages over other stabilization systems. For example, when properly installed, spinal stabilization system 10 may minimize torsional and shear stresses that tend to delaminate an intervertebral disc at a first vertebral level and may further protect an adjacent level from stresses, which may eliminate or reduce adjacent level disc degeneration. Spinal stabilization system 10 may also be able to provide superior pain relief by restricting undesirable spine motion by providing stability in lateral bending, torsion and shear, while simultaneously allowing movement in flexion-extension. Embodiments of spinal stabilization system 10 may be used in minimally invasive surgery (MIS) procedures as well as non-MIS procedures. It is believed that the ability to implant spinal stabilization system 10 using MIS procedures can provide additional advantages. MIS procedures seek to reduce cutting, bleeding, and tissue damage or disturbance associated with implanting a spinal implant in a patient's body. Exemplary procedures may use a percutaneous technique for implanting longitudinal rods and coupling elements. Examples of MIS procedures and related apparatus are provided in U.S. Patent Application Serial No. 10/698,049, filed October 30, 2003, U.S. Patent Application Serial No. 10/698,010, filed October 30, 2003, and U.S. Patent Application Serial No. 10/697,793, filed October 30, 2003, each of which is fully incorporated herein by reference.

The connection member can have an irregular surface that is produced on the connecting portion of the first rigid section and the connecting portion of the second rigid section by the method of: on a surface portion of the connecting portion of the first rigid section and the second rigid section that is to be joined to the flexible section, masking the surface portion in a random pattern with a maskant material, such that less than the entire surface portion is covered thereby; etching the surface portion such that first rigid section material or second rigid section material is removed thereby in areas uncovered by the maskant material, and areas covered by the maskant material are left intact; removing the maskant material; and repeating the masking, etching and removing steps upon the surface portion until a desired surface irregularity is achieved.

The connection member may have a flexible section that is joined to the first rigid section and the second rigid section by molding a material to contact the exterior surface of the connecting portion of the first rigid section and exterior surface of the connecting portion of the second rigid section.

The connection member may comprise a contact point between the molded material of the flexible section and the increased surface area of the first rigid section or the second rigid section that inhibits ingress of contaminants between the first rigid section and the flexible section or the second rigid section and the flexible section.

A contact point between the molded material of the flexible section and the increased surface area of a rigid section may inhibit separation of the first rigid section from the flexible section or the second rigid section from the flexible section.

In the foregoing specification, specific embodiments have been described with reference to the accompanying drawings. However, as one skilled in the art can appreciate, embodiments of the connection rod disclosed herein can be modified or otherwise implemented in many ways without departing from the spirit and scope of the disclosure. Accordingly, this description is to be construed as illustrative only and is for the purpose of teaching those skilled in the art the manner of making and using embodiments of a connection member having a flexible section between two rigid sections. It is to be understood that the embodiments shown and described herein are to be taken as exemplary. Equivalent elements or materials may be substituted for those illustrated and described herein. Moreover, certain features of the disclosure may be utilized independently of the use of other features, all as would be apparent to one skilled in the art after having the benefit of this description of the disclosure.

## Claims

1. A connection member for stabilization of the spine, comprising:
a first rigid section comprising:
a first portion; and
a connecting portion, wherein the connecting portion of the first rigid section has an exterior surface, wherein at least a portion of the exterior surface has an increased surface area;
a second rigid section comprising:
a first portion; and
a connecting portion, wherein the connecting portion of the second rigid section has an exterior surface, wherein at least a portion of the exterior surface is adapted to have an increased surface area; and
a flexible section joined at a first end to the connecting portion of the first rigid section and joined at a second end to the connecting portion of the second rigid section,
wherein separation of the flexible section from the first and second rigid sections is inhibited by the increased surface area of the exterior surface of the connecting portion of the first rigid section and the increased surface area of the exterior surface of the connecting portion of the second rigid section.

2. The connection member of claim 1, wherein each connecting portion of the first and second rigid sections has an irregular surface that constitutes the portion of the exterior surface having an increased surface area.

3. The connection member of claim 2, wherein the irregular surfaces of the connecting portions of the first rigid section and the second rigid section comprise a plurality of randomly sized, as defined by height and thickness, and spaced, as defined by proximity therebetween, protrusions which are substantially unstressed with respect to the exterior surface and which are constructed monolithically therefrom.

4. The connection member of claim 1, wherein the first rigid section and the second rigid section each comprises a metal from the set of metals including titanium, titanium alloys, stainless steel and steel alloys.

5. The connection member of claim 1, wherein the connecting portion of the first rigid section or the connecting portion of the second rigid section comprises one or more features for increased surface area, wherein each feature extends radially outward or recesses radially inward from the exterior surface of the connecting portion of the first rigid section or the connecting portion of the second rigid section, in particular wherein the increased surface area comprises one or more of an aperture, an undercut, a protrusion and a boss.

6. The connection member of claim 5, wherein the connecting portion has a tapered section.

7. The connection member of claim 1, wherein the flexible section material comprises polycarbonate urethane.

8. The connection member of claim 1, further comprising a first anchor member for anchoring the connection member to a first vertebra and a second anchor member for anchoring the connection member to a second vertebra.

9. A method of forming a connection member, comprising:
forming a first rigid section to have a first portion and a connecting portion, wherein the connecting portion has an exterior surface, wherein at least a portion of the exterior surface is formed to have an increased surface area;
forming a second rigid section to have a first portion and a connecting portion, wherein the connecting portion has an exterior surface, wherein at least a portion of the exterior surface is formed to have an increased surface area; and
molding a material about the connecting portion of the first rigid section and the connecting portion of the second rigid section to form a flexible section to form a flexible section between the first rigid section and the second rigid section.

10. The method of claim 9, wherein forming the at least a portion of the exterior surface of the first rigid section and the at least a portion of the second rigid section comprises forming an irregular surface portion by the method of:
masking the exterior surface in a random pattern with a maskant material, such that less than the entire exterior surface is covered thereby;
etching the surface portion such that first rigid section material or second rigid section material is removed thereby in areas uncovered by the maskant material, and areas covered by the maskant material are left intact;
removing the maskant material; and
repeating the masking, etching and removing steps upon the surface portion until a desired surface irregularity is achieved.

11. The method of claim 9, wherein forming at least a portion of the exterior surface of the first rigid section and forming at least a portion of the exterior surface of the second rigid section to have an increased surface area comprises forming one or more of an aperture, an undercut, a protrusion and a boss.

12. The method of claim 9, wherein the first rigid section is positioned at some angle relative to the second rigid section, wherein the connection member comprises a bend formed by the flexible section.

13. The method of claim 9, wherein forming at least a portion of the exterior surface of the first rigid section and forming at least a portion of the exterior surface of the second rigid section to have an increased surface area comprises forming exterior surfaces that comprise a plurality of randomly sized, as defined by height and thickness, and spaced, as defined by proximity there between, protrusions which are substantially unstressed with respect to the exterior surface and which are constructed monolithically therefrom.

14. The method of claim 9, wherein molding the flexible section comprises molding t he flexible section using a polycarbonate urethane material.

15. A spine stabilization system, comprising:
a connection member, comprising:
a first rigid section comprising:
a first portion; and
a connecting portion, wherein the connecting portion of the first rigid section has an exterior surface, wherein at least a portion of the exterior surface has an increased surface area;
a second rigid section comprising:
a first portion; and
a connecting portion, wherein the connecting portion of the second rigid section has an exterior surface, wherein at least a portion of the exterior surface has an increased surface area; and
a flexible section joined to the connecting portion of the first rigid section and further joined to the connecting portion of the second rigid section by molding a material to contact the exterior surface of the connecting portion of the first rigid section and the second rigid section,
wherein separation of the flexible section from the first and second rigid sections is inhibited by the increased surface area of the exterior surface of the connecting portion of the first rigid section and the increased surface area of the exterior surface of the connecting portion of the second rigid section; and
two or more anchoring members for affixing the first rigid portion and the section rigid portion of the connection member to two or more vertebrae.
